**Europäisches Patentamt**

**European Patent Office**

**Office européen des brevets**

(19)

(11) Veröffentlichungsnummer : **0 348 692 B1**

(12)

# EUROPÄISCHE PATENTSCHRIFT

(45) Veröffentlichungstag der Patentschrift :
**13.05.92 Patentblatt 92/20**

(51) Int. Cl.$^5$ : **A61M 25/00**

(21) Anmeldenummer : **89110097.6**

(22) Anmeldetag : **03.06.89**

(54) **Vorrichtung zum transvenösen oder arteriellen Einführen mittels eines Führungsdrahtes.**

(30) Priorität : **28.06.88 DE 3825631**

(43) Veröffentlichungstag der Anmeldung :
**03.01.90 Patentblatt 90/01**

(45) Bekanntmachung des Hinweises auf die
Patenterteilung :
**13.05.92 Patentblatt 92/20**

(84) Benannte Vertragsstaaten :
**AT CH DE FR GB LI NL SE**

(56) Entgegenhaltungen :
\* **Keine Entgegenhaltungen** \*

(73) Patentinhaber : **Osypka, Peter, Dr. Ing.**
**Basler Strasse 109**
**W-7889 Grenzach-Wyhlen (DE)**

(72) Erfinder : **Osypka, Peter, Dr. Ing.**
**Basler Strasse 109**
**W-7889 Grenzach-Wyhlen (DE)**

(74) Vertreter : **Patentanwälte Dipl.-Ing. Hans**
**Schmitt Dipl.-Ing. Wolfgang Maucher**
**Dreikönigstrasse 13**
**W-7800 Freiburg i.Br. (DE)**

EP 0 348 692 B1

## Beschreibung

Die Erfindung betrifft eine Vorrichtung zum transvenösen oder arteriellen Einführen von medizinischen Kathetern, Elektroden od.dgl. mittels eines Führungsdrahtes od.dgl.

Beim Einführen solcher Geräte in Adern und Gefäße bestehen immer wieder Schwierigkeiten, weil diese Gefäße bei relativ kleinem Querschnitt zum Teil enge Biegungen aufweisen, an denen z.B. Schraubwendeln od.dgl. sich verhaken können. Es wurden deshalb vor allem für Elektroden Schraubwendeln bekannt, die während des Einführens zurückgezogen sind und in Gebrauchsstellung mit einem entsprechend aufwendigen Mechanismus aus der Stirnseite der Elektrode ausgefahren und herausgeschraubt werden können. Dies führt allerdings zu einem entsprechend aufwendigen Elektrodenkopf, der zumindest über eine gewisse axiale Ausdehnung steif ist.

Es gibt aber oft Venen oder Arterien, die anatomisch so gebogen sind oder durch krankhafte Veränderungen so verengt sind, daß eine leichte Einführung eines Katheters oder einer Elektrode durch diese Gefäße nicht möglich oder nur mit großen Schwierigkeiten verbunden ist. Es kann dabei sogar passieren, daß Elektroden beschädigt oder unbrauchbar werden.

Es besteht deshalb die Aufgabe, eine Vorrichtung der eingangs erwähnten Art zu schaffen, die mit Hilfe eines einführbaren Führungsdrahtes, der auch vorgebogen sein kann, eine gute Einführung und Lokalisierung eines gewünschten Ortes in einem Hohlorgan, beispielsweise im Herzen oder auch im Kreislauf ermöglicht.

Zwar sind Führungsdrähte für diesen Zweck bereits bekannt, jedoch bedingen sie bisher, daß das einzuführende Teil dann seinerseits eine entsprechende Öffnung od.dgl. hat, die über diesen Führungsdraht paßt und somit den einzuführenden Katheter oder die Elektrode wiederum aufwendiger und mindestens bereichsweise steifer macht.

Die Erfindung sieht demgegenüber zur Lösung der Aufgabe vor, daß die Vorrichtung einen auf dem Führungsdraht gleitenden separaten Schlitten aufweist und daß dieser Schlitten mit wenigstens einer Aufnahme für das vordere Ende des einzuführenden Teiles versehen ist.

Dadurch können die Vorteile einer Einführung mit Hilfe eines Führungsdrahtes und der dadurch zu erreichenden genauen Lokalisierung verwirklicht werden auch bei solchen Kathetern oder Elektroden, die keine speziellen Gegenführungen für diesen Führungsdraht benötigen. Die Gegenführung wird vielmehr gewissermaßen von dem separaten Schlitten gebildet, der bei dem Einführvorgang zweckmäßigerweise vor der Stirnseite des Katheters oder der Elektrode angeordnet ist und somit auch eventuelle Vorsprünge oder scharfe Kanten oder Schraubwendeln abdeckt. Entweder kann die Vorwärtsbewegung dabei durch Schubkräfte an dem Katheter oder der Elektrode selbst oder aber auch an einem zusätzlichen Mandrin, der im Inneren einer solchen Elektrode liegen kann, erfolgen. Dabei ergibt sich der weitere Vorteil, daß dieser Schlitten auch zum Transport von Medikamentenkapseln dienen kann, da der Schlitten auch nur mit einem Mandrin vorwärtsbewegt werden kann. Solche sich nach dem Einführen öffnende oder auflösende Medikamentenkapseln sind an sich bekannt. Die Erfindung ermöglicht nun einen gezielten Transport zu einer zuvor durch den Führungsdraht genau lokalisierten Stelle, so daß ein entsprechendes Medikament nicht mehr überdosiert, sondern genau bedarfsweise bemessen sein kann.

Zweckmäßig ist es, wenn der Schlitten wenigstens eine Durchgangs- oder Führungsöffnung für den Führungsdraht sowie als Aufnahme eine Ausnehmung zum Umschließen des vordersten Endes bzw. der Stirnseite des Katheters, der Elektrode, eines Mandrins oder eines weiteren Führungsdrahtes hat. Dadurch ergibt sich eine zumindest in Einschubrichtung formschlüssige Kupplung zwischen Schlitten und einzuführendem Teil.

Dabei kann die Aufnahme-Ausnehmung ein Sackloch sein, in welches beispielsweise eine Schraubwendel eines Elektrodenkopfes paßt.

Der Führungsdraht kann an seinem distalen Ende eine Verdickung, insbesondere eine Kugel, haben, deren Querschnitt den Querschnitt der über den Führungsdraht greifenden Führungsöffnung des Schlittens übertrifft. Dadurch wird verhindert, daß der Schlitten am distalen Ende von dem Führungsdraht abgestreift werden kann. Außerdem kann auf diese Weise der Schlitten zusammen mit dem Führungsdraht aus dem Gefäß zurückgezogen werden.

Es ist aber auch - insbesondere zusätzlich - möglich, daß an dem Schlitten wenigstens ein Zugelement angreift, insbesondere an seinem in Einführrichtung rückwärtigen Ende. Damit könnte auch durch eine begrenzte Länge dieses Zugelementes verhindert werden, daß der Schlitten von dem distalen Ende des Führungsdrahtes gleitet, jedoch ist der Hauptvorteil eines solchen zusätzlichen Zugelementes darin zu sehen, daß der Schlitten zurückgezogen werden kann, ohne den Führungsdraht gleichzeitig zurückziehen zu müssen, so daß er für weitere Zwecke zunächst in seiner Lage bleiben kann. In jedem Falle wird durch ein zusätzliches Zugelement an dem Schlitten die Sicherheit erhöht.

Ausgestaltungen des Schlittens, die vor allem seine Einführung oder Rückziehung aufgrund einer günstigen Formgebung verbessern, sind Gegenstand der Ansprüche 6 und 7.

2

Die Aufnahme-Ausnehmung ist zweckmäßigerweise parallel zu der Führungsöffnung des Schlittens angeordnet. Einerseits findet sie so am meisten Platz und andererseits können dann die evtl. an dieser Aufnahme-Ausnehmung ausgeübten Schiebekräfte besonders gut in eine Vorwärtsbewegung mit Hilfe der Führung des Schlittens umgesetzt werden.

Eine Ausgestaltung der Aufnahme-Ausnehmung kann darin bestehen, daß sie den gesamten Schlitten in dessen Längsrichtung oder Vorschubrichtung durchsetzen und eine Schraubwendel od.dgl. in ihrem Innern klemmend oder mittels eines Gewindes während des Einführens axial festlegen kann, wobei die Schraubwendel aus dem vorderen Ende dieser Aufnahme-Ausnehmung in eine Wandung einer Körperhöhle od.dgl. einschraubbar ist. Beim Zurückziehen des Schlittens kann dann auch die Aufnahme-Ausnehmung über die mit der Schraubwendel festgelegte Elektrode od.dgl. zurückgleiten.

Eine weitere Möglichkeit besteht darin, daß die Aufnahme-Ausnehmung im Inneren des Schlittens abgestuft ist und ein erster Bereich einen größeren Querschnitt und ein in Einführrichtung am vorderen Ende des Schlittens mündender zweiter Bereich dieser Aufnahme-Ausnehmung einen kleineren Querschnitt hat. Somit könnte sich an der Abstufung ein Katheter oder Schlauch abstützen, durch welchen nach dem Vorschieben des Schlittens ein weiteres Teil einführbar ist.

Weitere Ausgestaltungen der Führungs- und Aufnahme-Öffnungen sowie des zweckmäßigen Werkstoffes des Schlittens oder auch einer Auskleidung der Öffnung sind Gegenstand der Ansprüche 11 bis 14. Vor allem die Verwendung eines aus Metall bestehenden Schlittens hat den Vorteil, daß sein Vorschub auf dem Röntgenschirm beobachtet werden kann. Gerade dann ist es aber vorteilhaft, wenn die Führungsöffnung mit gleitfähigem Kunststoff ausgekleidet ist.

Damit nach dem Einführen einer Elektrode oder eines Katheters mit Hilfe des Schlittens diese beiden Teile einfach z.B. durch eine Drehbewegung des Operateurs an dem eingeführten Teil voneinander gelöst und getrennt werden können, ist es vorteilhaft, wenn der Schlitten mit seiner Führung drehfest kuppelbar ist, insbesondere zwei parallele Führungsöffnungen für den gleichzeitigen Angriff an zwei Führungsdrähten aufweist und in dem außerhalb dieser Führungsöffnungen befindlichen Bereich die Aufnahme-Ausnehmung hat.

Eine zusätzliche oder andere Möglichkeit zur Erleichterung des Trennens des Schlittens von dem eingeführten Teil kann darin bestehen, daß der Schlitten und/oder der Führungsdraht eine Trennhilfe-Einrichtung zum Festlegen des Schlittens am distalen Ende des Führungsdrahtes hat, die eine der Zugkraft beim Auskuppeln eines Elektrodenkopfes entgegengesetzte und diese übertreffende Haltekraft hat. Möglichkeiten und Ausgestaltungen einer solchen Trennhilfe, die eine Verrastung oder Kupplung des Schlittens am distalen Ende des Führungsdrahtes erlaubt oder aber dem Operateur eine Gegenhaltekraft auf den Schlitten ermöglicht, sind Gegenstand der Ansprüche 17 bis 21.

Eine weitere Möglichkeit der Erleichterung des Auskuppelns einer Elektrode oder auch eines Katheters aus dem Schlitten kann darin bestehen, daß die Aufnahme-Ausnehmung auf ihrer der Führungsöffnung abgewandten Seite auch seitlich offen ist und die innere in Vorschubrichtung von dem Elektrodenkopf oder Katheterende beaufschlagte Wandung dieser Aufnahme-Ausnehmung gegen diese seitliche Öffnung in Vorschubrichtung schräg verläuft und bei Druck auf die Elektrode oder den Katheter den Elektrodenkopf oder das Katheterende nach außen ablenkt. Ebenso ließe sich an einer solchen seitlich offenen Aufnahme-Ausnehmung mit schräger Stirnwand eine Medikamentenkapsel od.dgl. durch einen Mandrin gut auswerfen.

Ausgestaltungen einer solchen Aufnahme-Öffnung in dem Sinne, daß während des Schlitten-Vorschubes die Elektrode, der Katheter, ein Mandrin od.dgl. genügend sicher gehalten sind, sind Gegenstand der Ansprüche 23 und 24.

Insgesamt ergibt sich durch diesen auf einem oder mehreren Führungsdrähten verstellbaren Schlitten die Möglichkeit, auch schwierige Einführungen von Kathetern oder Elektroden durchzuführen und deren Ende durch die vorher erfolgte genaue Lokalisierung des distalen Endes des Führungsdrahtes entsprechend präzise an ihr Ziel z.B. im Inneren des Herzens zu bringen. Somit kann auch die Operationszeit verkürzt werden, da die Zahl der Lokalisierungsversuche vermindert wird. Gleichzeitig können vor allem Elektroden mit vorteilhaften Schraubwendeln zum Fixieren eingeführt werden, ohne daß es aufwendiger Maßnahmen an diesen Schraubwendeln während ihres Einführens bedarf, da sie von dem Schlitten umschlossen sind und somit keine Verletzungen der Gefäße bewirken können, selbst wenn diese enge Krümmungen haben.

Nachstehend ist die Erfindung mit ihren ihr als wesentlich zugehörenden Einzelheiten anhand der Zeichnung in mehreren Ausführungsbeispielen näher beschrieben.

Es zeigt in z.T. schematisierter Darstellung und in vergrößertem Maßstab:

Fig. 1 eine Seitenansicht einer erfindungsgemäßen Vorrichtung mit einem Führungsdraht, einem darauf verschiebbaren Schlitten und einer mit einer Schraubwendel in eine Aufnahmeausnehmung dieses Schlittens eingreifende Elektrode nahe dem distalen Ende des Führungsdrahtes,

Fig. 2 eine der Fig. 1 entsprechende Vorrichtung, bei welcher der Führungsdraht eine Vorbiegung hat, die in einer Körperhöhle zur genauen Lokalisierung dient und zu einer selbsttätigen Trennung des Schlittens von dem Elektrodenkopf in diesem Biegungsbereich führt,

Fig. 3 eine abgewandelte Vorrichtung, bei welcher die Aufnahme-Ausnehmung an dem Schlitten durchgehend ist und zusätzlich an dem Schlitten ein Zugelement angreift,

Fig. 4 eine Vorrichtung, bei welcher der Führungsdraht mit einer Schraubwendel fixierbar ist oder als Elektrode ausgebildet ist und an dem Schlitten ein Katheter angreift,

Fig. 5 eine Stirnsansicht eines Führungsschlittens mit zwei Führungsöffnungen,

Fig. 6 eine Seitenansicht und

Fig. 7 eine Draufsicht des Schlittens gemäß Fig. 5, welcher an zwei parallelen Drähten geführt ist, die eine Verdrehung des Schlittens beim Herausschrauben und Herausdrehen des Elektrodenkopfes aus der Aufnahme-Öffnung verhindern,

Fig. 8 eine der Fig. 1 entsprechende Darstellung, bei welcher an der in Einführrichtung vorderen Seite des Schlittens eine hakenförmige Trennhilfe zum Verrasten des Schlittens an dem distalen Ende des Führungsdrahtes beim kurzen Zurückziehen der Elektrode zu ihrem Heraustrennen aus dem Schlitten vorgesehen ist,

Fig. 9 und 10 eine gegenüber Fig. 8 abgewandelte Trennhilfe, bei welcher ein widerhakenartiger Vorsprung oder Ring an dem Führungsdraht mit Abstand zu dem Ende des Führungsdrahtes angeordnet ist,

Fig. 11 eine Ausführungsform, bei welcher als Trennhilfe an dem Schlitten ein den Führungsdraht umschließender Schlauch od.dgl Gegenhalter angreift,

Fig. 12 einen Schlitten mit einer in seinem Inneren befindlichen, bei Nichtgebrauch in die Führungsöffnung ragenden, durch den Führungsdraht daraus verdrängbaren Haltefeder und

Fig. 13. die Verrastung dieser Haltefeder an einem im Querschnitt dünneren distalen Ende des Führungsdrahtes,

Fig. 14 eine Ausführungsform, bei welcher die Aufnahme-Ausnehmung auch seitlich offen ist und eine schräge Stirnwand zum Auskuppeln durch erhöhten Druck auf die Elektrode od.dgl. hat, wobei innerhalb der Ausnehmung eine Halteklemme für den Elektrodenkopf od.dgl. vorgesehen ist,

Fig. 15 eine Draufsicht des Schlittens und seiner seitlich offenen Aufnahme-Ausnehmung mit der Halteklemme sowie

Fig. 16 eine gegenüber Fig. 15 abgewandelte Ausführungsform und

Fig. 17 eine Draufsicht des Schlittens gemäß Fig. 16 mit der seitlichen Öffnung der Aufnahme-Ausnehmung, die bereichsweise verengt ist.

Eine in unterschiedlichen Ausführungsformen realisierbare, in den vorstehend aufgezählten Figuren dargestellte Vorrichtung dient zum transvenösen oder arteriellen Einführen von medizinischen Elektroden 1 oder Kathetern 2 mit Hilfe eines Führungsdrahtes 3. Dabei ist bei allen Ausführungsbeispielen als wichtigstes Element dieser Vorrichtung ein auf dem Führungsdraht 3 gleitender oder gleitfähiger Schlitten 4 vorgesehen, welcher wenigstens eine Aufnahme 5 für das vordere Ende des einzuführenden Teiles hat. Statt das vordere Ende einer Elektrode 1 oder eines Katheters 2 an dem Schlitten 4 angreifen zu lassen, könnte er auch mit Hilfe eines Mandrins verschoben werden und zum Transport von Medikamenten dienen.

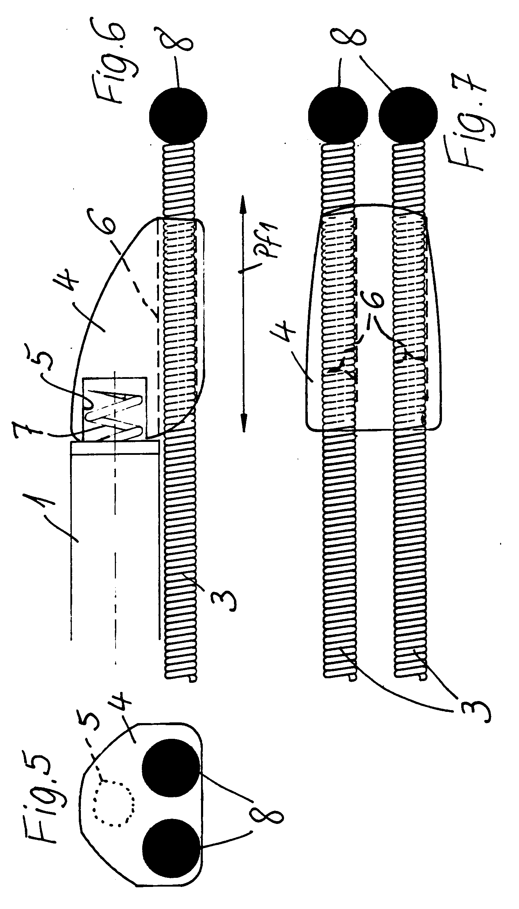

Der Schlitten 4 hat wenigstens eine - im Ausführungsbeispiel gemäß den Figuren 5 bis 7 zwei - Durchgangs- oder Führungöffnung 6 für den Führungsdraht 3 sowie als Aufnahme eine Ausnehmung 5 zum Umschließen des vordersten Endes bzw. der Stirnseite des Katheters 2, der Elektrode 1, eines Mandrins oder auch eines weiteren Führungsdrahtes.

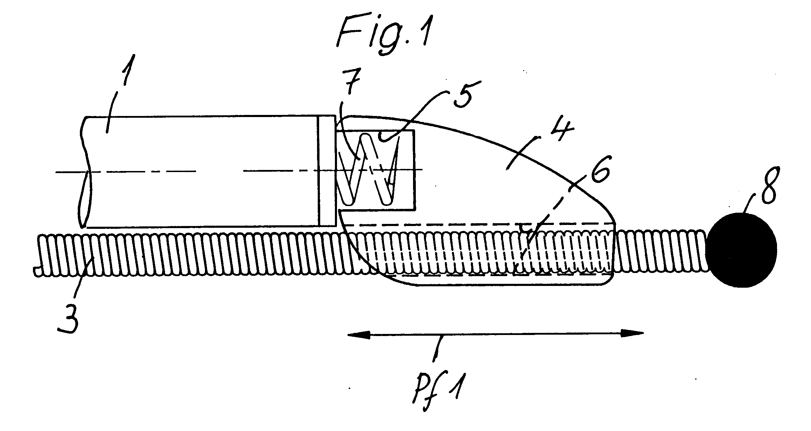

In den Ausführungsbeispielen gemäß den Figuren 1, 2, 6 und 8 bis 13 ist die Aufnahme-Ausnehmung 5 ein Sackloch, in welches beispielsweise eine Schraubwendel 7 eines Elektrodenkopfes paßt.

Der Führungsdraht 3 hat an seinem distalen Ende eine Verdickung 8, insbesondere eine Kugel od.dgl., deren Querschnitt den Querschnitt der über den Führungsdraht 3 greifenden Führungsöffnung 6 des Schlittens 4 übertrifft. Somit ist ausgeschlossen, daß der Schlitten 4 von dem Führungsdraht 3 abgestreift werden kann. Ferner kann auf diese Weise der Schlitten 4 zusammen mit dem Führungsdraht 3 aus einem Gefäß zurückgezogen werden.

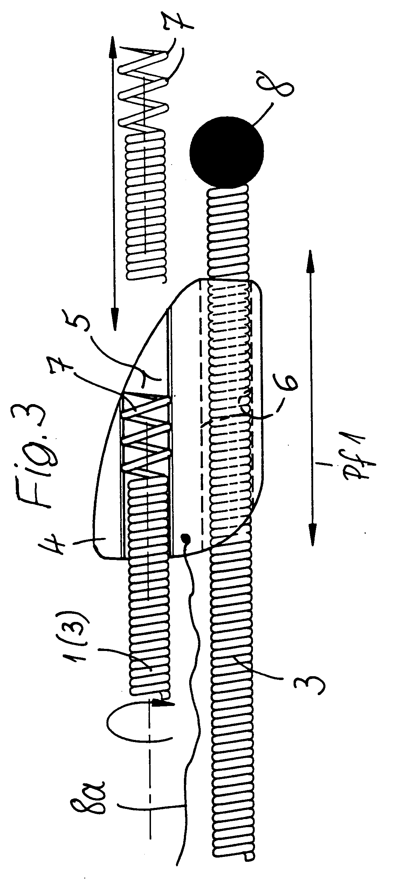

In Fig. 3 ist angedeutet, daß an dem Schlitten 4 wenigstens ein Zugelement 8a,z.B. ein Faden, angreifen kann und zwar insbesondere an seinem in Einführrichtung rückwärtigen Ende. Dies erhöht die Sicherheit dafür, daß der Schlitten 4 in jedem Falle zurückgezogen werden kann und ermöglicht es, den Schlitten 4 auch ohne Zurückziehung des Führungsdrahtes 3 zurückzuziehen und den Führungsdraht 3 noch für eine weitere Verwendung liegenzulassen.

In allen Ausführungsbeispielen erkennt man, daß der Schlitten 4 sich in Einführrichtung von der Aufnahme 5 gegen die vordere Münderung der Führungsöffnung 6 hin verjüngt und daß das rückwärtige Ende des Schlittens 4 im Bereich des Eintrittes der Führungsöffnung 6 und vorzugsweise auch der Aufnahme 5 abgerundet, evtl. abgeschrägt oder verjüngt ist, so daß die Hin- und Herbewegungen des Schlittens gemäß dem Doppelpfeil z.B.Pf1 in Fig.1 erleichtert sind. Die Aufnahme-Ausnehmung 5 ist dabei parallel zu der Führungsöffnung 6 des Schlittens 4 angeordnet, damit die in dieser Aufnahme-Ausnehmung 5 ausgeübten Vorschubkräfte möglichst nicht zu Verkantungen zwischen Führungsöffnung 6 und Führungsdraht 3 führen.

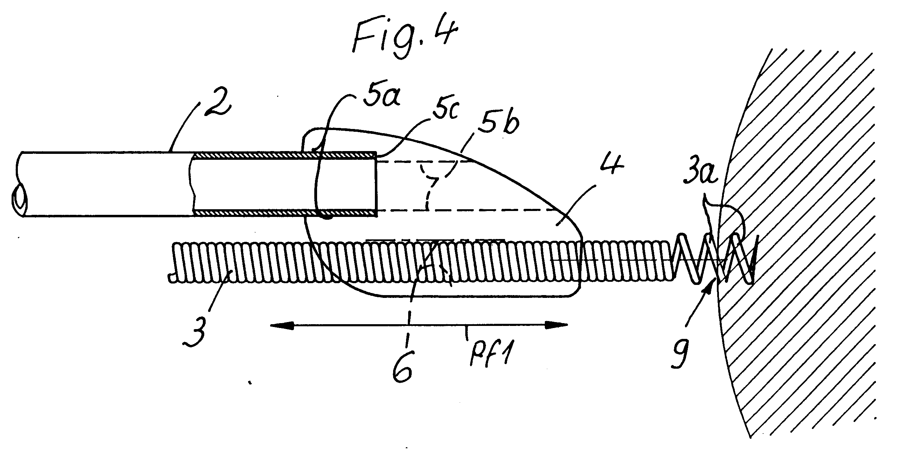

In den Figuren 3 und 4 erkennt man Ausführungsbeispiele, bei denen die Aufnahme-Ausnehmung 5 den gesamten Schlitten 4 in dessen Längsrichtung oder Vorschubrichtung durchsetzt und eine Schraubwendel 7 od.dgl. in ihrem Inneren klemmend oder mittels eines Gewindes während des Einführens axial festlegt, wobei die Schraubwendel 7 gemäß Fig. 3 aus dem vorderen Ende dieser Aufnahme-Ausnehmung 5 beispielsweise in eine Wandung einer Körperhöhle od.dgl. einschraubbar ist. Da hinter dieser Schraubwendel 7 die Elektrode einen verminderten Querschnitt hat, kann der Schlitten danach zurückgezogen werden, wobei dann diese durchgehende Aufnahme-Ausnehmung 5 ebenfalls als Führungsöffnung dient.

In Fig. 4 erkennt man, daß die Aufnahme-Ausnehmung 5 im Inneren des Schlittens 4 abgestuft sein kann und ein erster Bereich 5a einen größeren Querschnitt und ein in Einführrichtung am vorderen Ende des Schlittens 4 mündender zweiter Bereich 5b diese Aufnahme-Ausnehmung 5 einen kleineren Querschnitt haben kann. Somit kann an der zwischen den Bereichen 5a und 5b gebildeten Stufe 5c ein Katheter 2 abgestützt werden, durch welchen dann später beliebige Elektroden od.dgl. hindurchgeführt werden können, da diese durch den Bereich 5b aus dem Schlitten 4 austreten können. Es können dann also nachträglich Elektroden nahe dem Fixierungspunkt 9 des in diesem Falle ebenfalls eine Schraubwendel 3a aufweisenden Führungsdrahtes 3 angelegt werden.

Vor allem in den Ausführungsbeispielen gemäß den Figuren 3 und 4 können die Führungsöffnung 6 und die Aufnahme-Ausnehmung 5 einen im wesentlichen übereinstimmenden Querschnitt haben und unter Umständen sogar wechselweise zur Führung oder zur Aufnahme dienen.

Für eine gute Einführbarkeit ist es zweckmäßig, wenn der Schlitten 4 aus gleitfähigem Kunststoff und/oder körperverträglichem Metall, insbesondere Edelstahl besteht, wobei Metall den Vorteil hat, daß der Weg des Schlittens 4 auf dem Röntgenschirm verfolgt werden kann. Es ist dann günstig, wenn die Führungsöffnug 6 bzw. -öffnungen und/oder die Aufnahme-Ausnehmung 5 mit inertem, insbesondere gleitfähigem Kunststoff ausgegkleidet ist oder sind, weil dann trotzdem der Schlitten 4 gut über den Führungsdraht geschoben und ein einzuführendes Teil gut aus der Aufnahme-Ausnehmung herausgeführt werden können.

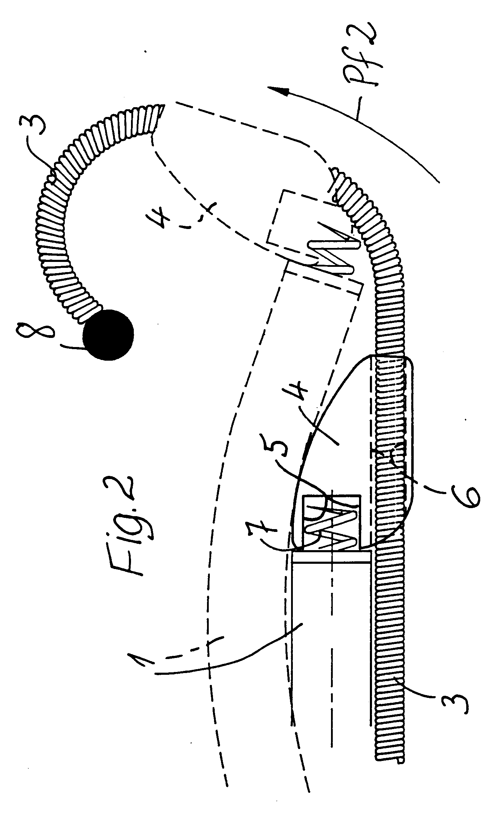

Wichtig ist es, daß nach dem Einführen eines einzuführenden Teiles dieses möglichst einfach und schnell wieder von dem Schlitten 4 gelöst werden kann. Fig. 2 zeigt eine praktisch automatische Entkupplung zwischen Schlitten 4 und Elektrode 1 in solchen Fällen, in denen der Führungsdraht eine vorgespannte Krümmung an seinem distalen Ende hat, die in solchen Fällen vorgesehen ist, in denen eine genaue Lokalisierung in einer Körperhöhle oder einem Organ, bevorzugt im Herzen durchgeführt werden soll. Solche J-förmig vorgebogenen Führungsdrähte sind bekannt. Gleitet nun der Schlitten in der in Fig. 2 dargestellten Weise auf diese Krümmung auf, wird er aus seiner bisherigen Vorschubrichtung gemäß dem Pfeil Pf2 ausgelenkt, so daß die den Schlitten 4 vorschiebende Stirnseite der Elektrode 1 und damit die Schraubwendel 7 aus dem Bereich der Aufnahmeöffnung 5 gelangen, wie es die gestrichelte Darstellung in Fig. 2 zeigt.

Eine andere Möglichkeit besteht natürlich vor allem bei einem Sackloch als Aufnahme-Ausnehmung 5 darin, das Elektrodenende aus dieser Öffnung rückwärts herauszudrehen. Um dies dem Benutzer zu erleichtern und zu verhindern, daß dabei der Schlitten 4 evtl. um seine Führungsöffnung 6 auf dem Führungsdraht 3 verschwenkt oder verdreht wird, kann der Schlitten 4 auf unterschiedliche Weise mit seiner Führung drehfest kuppelbar sein. Eine mögliche Lösung zeigen die Figuren 5 bis 7, in denen man zwei parallele Führungsöffnungen 6 für den gleichzeitigen Angriff an zwei Führungsdrähten 3 erkennt, wobei in dem außerhalb dieser Führungsöffnungen 6 befindlichen Bereich die Aufnahme-Ausnehmung 5 angeordnet ist. Es leuchtet ein, daß beim kurzen Zurückschrauben der Schraubwendel 7 der Elektrode 1 aus der Aufnahme-Ausnehmung 5 des Schlittens 4 heraus dieser sich auf keinen Fall in irgend einer Weise verdrehen läßt, da er an zwei parallelen Führungsdrähten 3 gehalten ist.

Vor allem für solche Fälle, bei denen das Trennen des eingeführten Teiles von dem Schlitten ausschließlich durch eine kurze Rückzugbewegung durchgeführt werden soll, können der Schlitten 4 und/oder der Führungsdraht 3 eine verschieden realisierbare Trennhilfe-Einrichtung haben, womit der Schlitten 4 am distalen Ende des Führungsdrahtes 3 mit einer solchen Haltekraft möglich ist, daß die dieser Haltekraft entgegengesetzte Zugkraft beim Auskuppeln des Elektrodenkopfes od.dgl. nicht nur entgegengesetzt, sondern auch übertroffen ist.

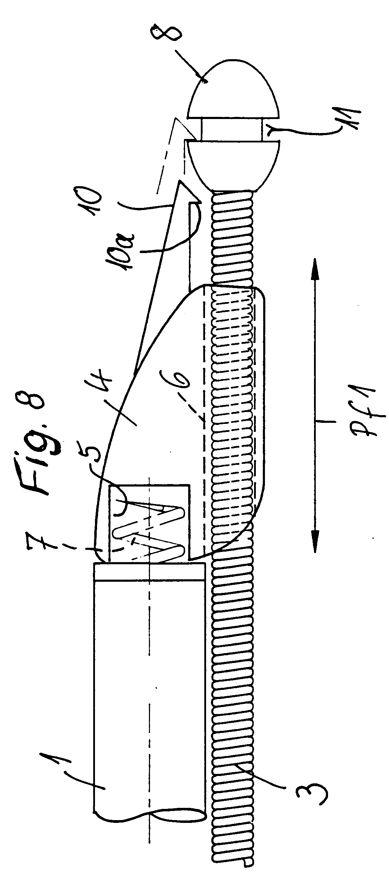

Fig. 8 zeigt ein Ausführungsbeispiel, bei welchem als Trennhilfe an dem Schlitten 4 ein Widerhaken 10 oder Kupplungsvorsprung vorgesehen ist, wobei der Widerhaken 10 einen in eine Ringnut 11 der kugeligen Verdickung 8 des Führungsdrahtes 3 einrastenden Hakenvorsprung 10a aufweist.

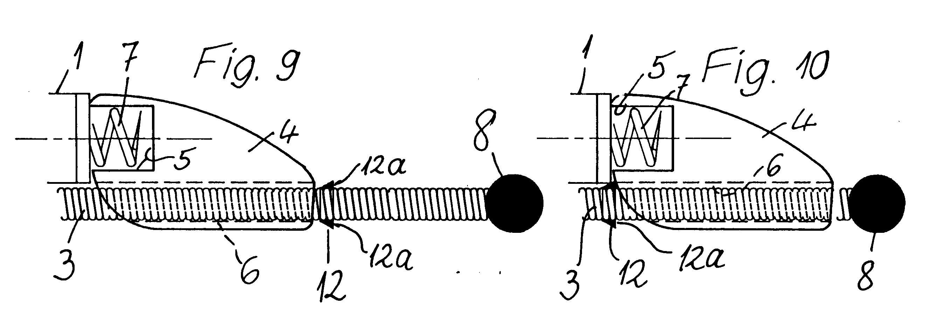

Fig. 9 und 10 zeigen ein Ausführungsbeispiel, bei welchem als Trennhilfe an dem Führungsdraht 3 ein Widerhaken 12 od. dgl. Kupplungsvorsprung vorgesehen ist, wobei dieser Widerhaken 12 des Führungsdrahtes 3 einen Anschlag 12a für den über ihn hinweggeschobenen Schlitten 4 mit Abstand zu der kugeligen Verdickung 8 aufweist. Dieser Anschlag 12a ist dabei zweckmäßigerweise ringförmig ausgebildet und sein Abstand von der kugeligen Verdickung 8 ist gleich oder größer der Gesamtlänge des Schlittens 4.

Die vorerwähnten widerhakenförmigen Trennhilfen 10 und 12 bestehen dabei zweckmäßigerweise aus Kunststoff, um gut zu federn und dennoch genügend Haltekraft zu erzeugen.

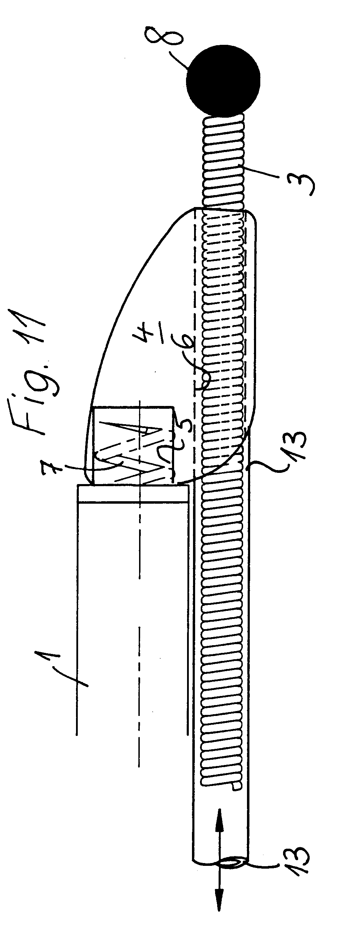

Wieder eine andere Möglichkeit zeigt Fig. 11, bei welcher als Trenn- oder Lösehilfe ein an dem in Einschubrichtung hinteren Ende des Schlittens 4 angreifender, über den Führungsdraht 3 passender Schlauch 13 vorgesehen ist, der mit dem Schlitten 4 insbesondere-verbunden, z.B. verklebt ist, so daß er sogar auch noch als Rückzughilfe dienen kann. Wird zunächst einmal die Elektrode 1 für ihre Trennung aus der Ausnehmung 5 etwas zurückgezogen, kann der Benutzer eine Druckkraft in Längsrichtung dieses Schlauches 13 ausüben und so verhindern, daß der Schlitten 4 der Rückzugbewegung der Elektrode 1 folgt. Ist die Elektrode 1 von dem Schlitten frei, kann sogar mit Hilfe des Schlauches durch eine Verdrehung des Schlittens 4 gut aus dem Stirnseiten-Bereich der Elektrode 1 herausgeschwenkt werden.

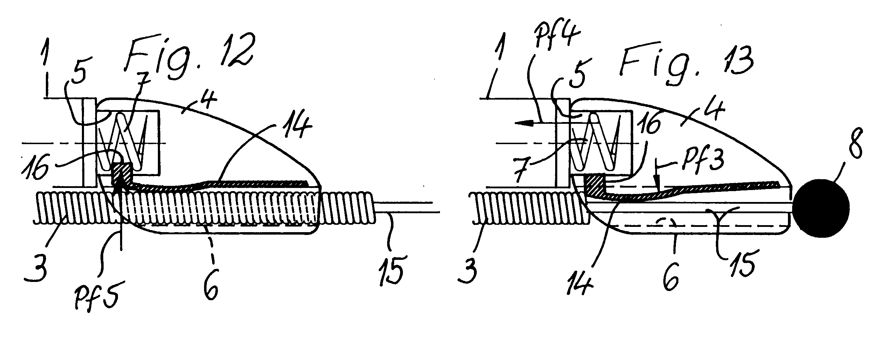

Im Ausführungsbeispiel gemäß den Figuren 12 und 13 ist als Trenn- oder Lösehilfe im Inneren des Schlittens 4 eine durch den Führungsdraht 3 aus der Führungsöffnung 6 in eine innerhalb des Schlittens befindliche Ausnehmung gegen eine Rückstellkraft ausschwenkbare Haltefeder 14, zweckmäßigerweise eine Blattfeder, vorgesehen, und am distalen Ende des Führungsdrahtes 3 ist eine Querschnittsverminderung 15 angeordnet, in welche diese Feder 14 bei Erreichen dieses Abschnittes gemäß Fig. 13 und entsprechend dem Pfeil Pf3 einrastet, wonach nun die Elektrode 1 und ihre Schraubwendel 7 gemäß dem Pfeil Pf4 zurückgezogen werden können, ohne daß der Schlitten 4 dieser Bewegung folgen kann. In vorteilhafter Weise kann dabei durch diese Trennhilfe zunächst sogar die Verankerung der Elektrode 1 an dem Schlitten 4 verbessert werden, indem diese als Trennhilfe im Inneren des Schlittens 4 angeordnete Blattfeder 14 an ihrem auslenkbaren Ende einen in Auslenkrichtung gemäß dem Pfeil Pf5 (Fig. 12) entgegen der Federkraft ragenden Vorsprung 16 hat, der bei ausgelenkter Feder gemäß Fig. 12 in diese Aufnahme-Ausnehmung 5 und dort in eine Ausnehmung des Elektrodenkopfes oder - wie dargestellt - zwischen die Windungen der Wendel 7 des Elektrodenkopfes paßt. Diese Trennhilfe hat somit eine Doppelfunktion, wobei ihre zweite Funktion ihrer Hauptfunktion sogar entgegengesetzt ist.

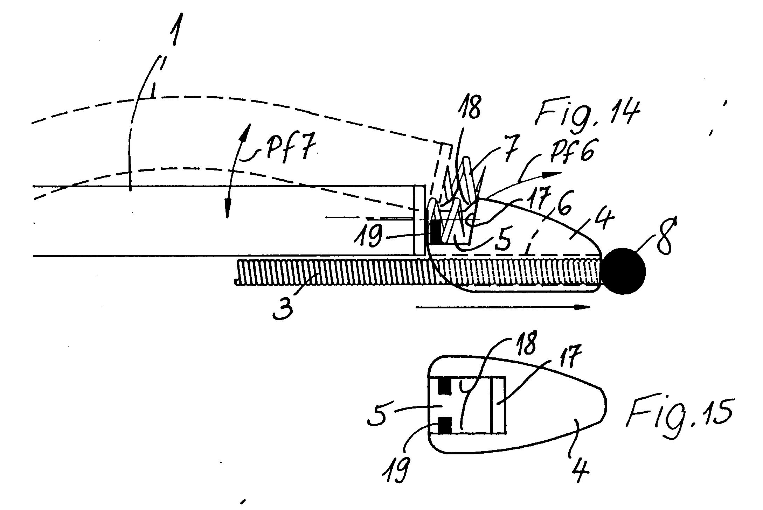

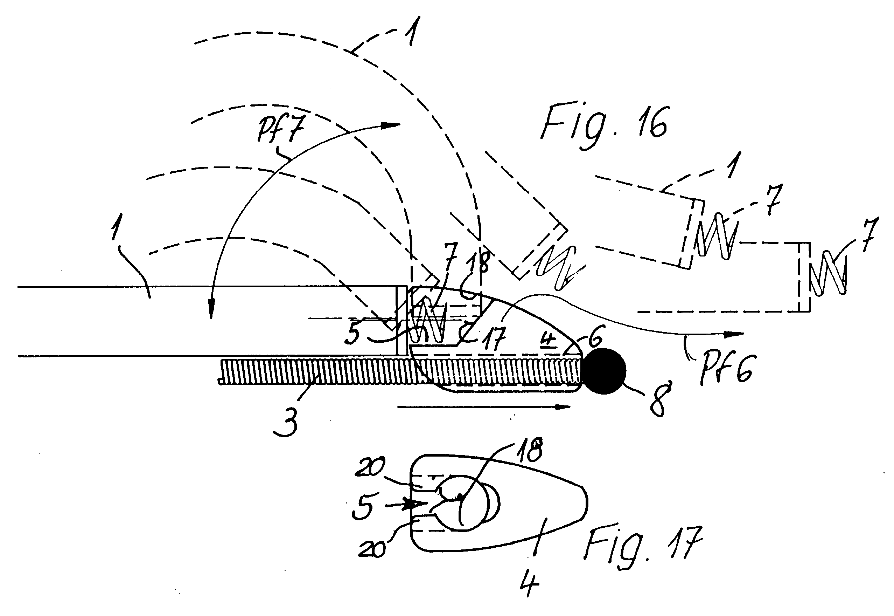

Die Figuren 14 bis 17 zeigen Möglichkeiten, wonach die Aufnahme-Ausnehmung 5 auf ihrer der Führungsöffnung 6 abgewandten Seite seitlich offen sind und die innere, in Vorschubrichtung von dem Elektrodenkopf oder Katheterende beaufschlagte Wandung 17 der Aufnahme-Ausnehmung 5 gegen diese seitliche Öffnung 18 in Vorschubrichtung schräg verläuft und bei axialem Druck auf die Elektrode 1 oder den Katheter 2 in Einführrichtung den Elektrodenkopf oder das Katheterende gemäß der gestrichelten Darstellung der Figur 14 und 16 ausbiegt und dabeinach außen ablenkt. Wird also über die Elektrode 1 oder den Katheter 2 in Richtung des Pfeiles Pf6 auch nach dem Anschlag des Schlittens 4 an der Verdickung 8 eine Druckkraft ausgeübt oder erhöht, führt dies automatisch zu dem seitlichen Herausgleiten aus der Aufnahme-Ausnehmung 5. In diesem Falle erfolgt also das Trennen nicht durch eine Rückzugbewegung des einzuführenden Teiles relativ zu dem Schlitten mit dem Erfordernis, den Schlitten dabei festzulegen, sondern die Trennung erfolgt durch eine zusätzliche Druckkraft in Einführrichtung.

Damit aber während des Einführens und insbesondere auch in gekrümmten Bereichen des Einführweges keine ungewollte Auskupplung des Elektroden- oder Katheterendes aus dem Schlitten 4 erfolgt, ist in beiden Ausführungsbeispielen vor allem gemäß Fig. 15 und 17 nahe dem der stirnseitigen Schrägfläche 17 abgewandten Ende der Aufnahme-Öffnung 5 wenigstens eine Klemme 19 (Fig. 14 und 15) oder Querschnittsverengung 20 (Fig. 16 und 17) zum Eingreifen in Ausnehmungen oder Zwischenräume einer Wendel 7 des Elektrodenkopfes oder auch des Katheters vorgesehen.

Bei der Lösung nach Fig. 16 und 17 übergreift die am Eintritt in die Aufnahme-Ausnehmung 5 vorgesehene Verengung 20 auch die seitliche Öffnung 18 der Aufnahme-Ausnehmung teilweise, so daß die seitliche Öffnung 18 von dem hinteren Ende des Schlittens 4 her zunächst eine schmale, sich dann erweiternde Umrißform hat und die Größe der Erweiterung wenigstens dem Umfang der Schraubwendel 7 oder des Elektroden- oder Katheterkopfes entspricht. Somit kann die Wendel 7 erst dann aus dieser seitlichen Öffnung 18 und ihrer Erweiterung austreten, wenn durch genügend großen axialen Druck sich der Katheter gemäß dem Bogenpfeil Pf7 in Fig. 16 soweit verformt hat, daß die Wendel 7 praktisch um 90° verschwenkt ist. Sowie die Wendel 7 aus der Aufnahme-Ausnehmung 5 seitlich herausgeglitten ist, stellt sich dann gemäß der gestrichelten Darstellung in Fig. 16 die Elektrode wieder axial ein, wobei sie gleichzeitig auch in axialer Richtung vorwärtsbewegt wird, so daß sie innerhalb einer Körperhöhle dann problemlos eingeschraubt werden kann.

Insgesamt ergibt sich durch den erfindungsgemäßen Schlitten eine problemlose Einführung und Plazierung von Elektroden im Ventrikel oder auch im Vorhof oder auch ein sicheres und problemloses Einführen eines Katheters. Dabei wird in vorteilhafter Weise bei einer Schraubelektrode mit feststehender Wendel diese geschützt, so daß Verletzungen der Venenwand vermieden werden.

Bei Einführung einer solchen Schraubelektrode mit Hilfe eines Schlittesn 4 in den rechten Ventrikel genügt es, wenn die Elektrode bis in den Ventrikel eingeführt, dort durch Linksdrehen des Schraubenkopfes (Fig. 1) vom Schlitten gelöst und dann an beliebiger Stelle des Ventrikels die Schraube eingedreht wird.

Es sei noch erwähnt, daß die Darstellung der Fig. 2 auch eine abgewandelte Arbeitsweise illustriert, bei welcher zunächst ein Führungsdraht 3 mit kugeliger Verdickung 8 plaziert wird, über den ein Schlitten 4 mit einem zweiten Führungsdraht geschoben wird, der an seinem distalen Ende eine kleine Schraubwendel hat. Hat man den Schlitten 4 in die distale Endposition geschoben, kann durch Weiterschrauben des als "Huckepack" mitgeführten zweiten Führungsdrahtes dieser in unmittelbarer Nähe der Verdickung 8 in das Gewebe hineingeschraubt werden. Durch Zurückziehen des Schlittens 4 zusammen mit dem ersten Führungsdraht 3 und dessen Verdickung 8 befindet sich dann im Herzen ein Fixierungspunkt mit Hilfe eines neuen eingeschraubten Führungsdrahtes, über welchen wiederum ein Schlitten plaziert werden kann, womit z.B. ein großlumiges Katheter eingeführt werden kann, wie es Fig. 4 zeigt.

Neben den verschiedenen vorerwähnten Anwendungsmöglichkeiten und Arbeitsweisen eröffnet der Schlitten 4 weitere vorteilhafte Möglichkeiten wie z.B. ein gezieltes Plazieren oder Zuführen eines Medikamentes im Inneren des Herzens oder auch eines sonstigen Hohlorganes, so daß durch diese gezielte Applikation dann auch entsprechend weniger Wirkstoff als bisher erforderlich wird.


**Patentansprüche**


1. Vorrichtung zum transvenösen oder arteriellen Einführen von medizinischen Kathetern (2), oder Elektroden (1) mittels eines Führungsdrahtes (3), **dadurch gekennzeichnet**, daß sie einen auf dem Führungsdraht (3) gleitenden separaten Schlitten (4) aufweist und daß dieser Schlitten (4) mit wenigstens einer Aufnahme (5) für das vordere Ende des einzuführenden katheders oder der einzuführenden Elektrode versehen ist.

2. Vorrichtung nach Anspruch 1, dadurch gekennzeichnet, daß der Schlitten (4) wenigstens eine Durchgangs- oder Führungsöffnung (6) für den Führungsdraht (3) sowie als Aufnahme eine Ausnehmung 5 zum Umschließen des vordersten Endes bzw. der Stirnseite des Katheters (2), der Elektrode (1), eines Mandrins oder eines weiteren Führungsdrahtes hat.

3. Vorrichtung nach Anspruch 1 oder 2, dadurch gekennzeichnet, daß die Aufnahme-Ausnehmung (5) ein Sackloch ist, in welches eine Schraubwendel (7) eines Elektrodenkopfes paßt.

4. Vorrichtung nach einem der Ansprüche 1 bis 3, dadurch gekennzeichnet, daß der Führungsdraht (3) an seinem distalen Ende eine Verdickung (8), insbesondere eine Kugel, hat, deren Querschnitt den Querschnitt der über den Führungsdraht (3) greifenden Führungsöffnung (6) des Schlittens (4) übertrifft.

5. Vorrichtung nach einem der Ansprüche 1 bis 4, dadurch gekennzeichnet, daß an dem Schlitten (4) wenigstens ein Zugelement (8a) angreift, insbesondere an seinem in Einführrichtung rückwärtigen Ende.

6. Vorrichtung nach einem der Ansprüche 1 bis 5, dadurch gekennzeichnet, daß der Schlitten (4) sich in Einführrichtung von der Aufnahme (5) für die einzuführende Elektrode oder Katheter gegen die vordere Mündung der Führungsöffnung hin verjüngt.

7. Vorrichtung nach einem der Ansprüche 1 bis 6, dadurch gekennzeichnet, daß das rückwärtige Ende des Schlittens (4) im Bereich des Eintrittes der Führungsöffnung (6) und vorzugsweise auch der Aufnahme (5) abegrundet, abgeschrägt oder verjüngt ist.

8. Vorrichtung nach einem der Ansprüche 1 bis 7, dadurch gekennzeichnet, daß die Aufnahme-Ausnehmung (5) parallel zu der Führungsöffnung (6) des Schlittens (4) angeordnet ist.

9. Vorrichtung nach einem der Ansprüche 1 bis 8, dadurch gekennzeichnet, daß die Aufnahme-Ausnehmung (5) den gesamten Schlitten (4) in dessen Längsrichtung oder Vorschubrichtung durchsetzt und eine Schraubwendel (7) od.dgl. in ihrem Inneren klemmend oder mittels eines Gewindes während des Einführens axial festlegt, wobei die Schraubwendel (7) aus dem vorderen Ende dieser Aufnahme-Ausnehmung (5) in eine Wandung einer Körperhöhle od.dgl. einschraubbar ist.

10. Vorrichtung nach einem der vorstehenden Ansprüche, dadurch gekennzeichnet, daß die Aufnahme-Ausnehmung im Inneren des Schlittens (4) abgestuft ist und ein erster Bereich (5a) einen größeren Querschnitt und ein in Einführrichtung am vorderen Ende des Schlittens (4) mündender zweiter Bereich (5b) dieser Aufnahme-Ausnehmung (5) einen kleineren Querschnitt hat.

11. Vorrichtung nach einem der Ansprüche 1 bis 10, dadurch gekennzeichnet, daß die Führungsöffnung und die Aufnahme-Ausnehmung jeweils kreisrunde, geschlossene Querschnitte haben.

12. Vorrichtung nach einem der vorstehenden Ansprüche, dadurch gekennzeichnet, daß die Führungsöffnung (6) und die Aufnahme-Ausnehmung (5) einen übereinstimmenden Querschnitt haben und wechselweise zur Führung oder zur Aufnahme dienen.

13. Vorrichtung nach einem der Ansprüche 1 bis 12, dadurch gekennzeichnet, daß der Schlitten (4) aus gleitfähigem Kunststoff und/oder körperverträglichem Metall, insbesondere Edelstahl besteht.

14. Vorrichtung nach einem der Ansprüche 1 bis 13, dadurch gekennzeichnet, daß die Führungsöffnung(en) (6) und/oder Aufnahme-Ausnehmung(en) mit inertem, insbesondere gleitfähigem Kunststoff ausgekleidet ist(sind).

15. Vorrichtung nach einem der Ansprüche 1 bis 14, dadurch gekennzeichnet, daß der Schlitten (4) mit seiner Führung drehfest kuppelbar ist, insbesondere zwei parallele Führungsöffnungen (6) für den gleichzeitigen Angriff an zwei Führungsdrähten (3) aufweist und in dem außerhalb diesen Führungsöffnungen (6) befindlichen Bereich die Aufnahme-Ausnehungen (5) hat.

16. Vorrichtung nach einem der Ansprüche 1 bis 15, dadurch gekennzeichnet, daß der Schlitten (4) und-/oder der Führungsdraht (3) eine Trennhilfe-Einrichtung zum Festlegen des Schlittens (4) am distalen Ende des Führungsdrahtes (3) hat, die eine der Zugkraft beim Auskuppeln des Elektrodenkopfes od.dgl. entgegengesetzte und diese übertreffende Haltekraft hat.

17. Vorrichtung nach einem der Ansprüche 1 bis 16, dadurch gekennzeichnet, daß als Trennhilfe an dem Führungsdraht (3) und/oder dem Schlitten (4) ein Widerhaken (10, 12) oder Kupplungsvorsprung vorgesehen ist, und daß der Widerhaken (12) des Führungsdrahtes (3) einen Anschlag (12a) für den über ihn hinweggeschobenen Schlitten (4) und/oder der an dem Schlitten selbst angeordnete Widerhaken (10) einen in eine Ringnut (11) der kugeligen Verdickung (8) des Führungsdrahtes (3) einrastenden Hakenvorsprung (10a) aufweist.

18. Vorrichtung nach einem der vorstehenden Ansprüche, dadurch gekennzeichnet, daß die widerhakenförmige Trennhilfe (10, 12) an dem Führungsdraht (3) und/oder an dem Schlitten (4) aus Kunststoff besteht.

19. Vorrichtung nach einem der Ansprüche 1 bis 18, dadurch gekennzeichnet, daß als Trenn- oder Lösehilfe ein an dem in Einschubrichtung hinteren Ende des Schlittens (4) angreifender, über den Führungsdraht (3) passender Schlauch (13) vorgesehen ist, der mit dem Schlitten (4) insbesondere verbunden, z.B. verklebt ist.

20. Vorrichtung nach einem der Ansprüche 1 bis 19, dadurch gekennzeichnet, daß als Trenn- oder Lösehilfe im Inneren des Schlittens (4) eine durch den Führungsdraht (3) aus der Führungsöffnung (6) in eine innerhalb des Schlittens befindliche Ausnehmung gegen eine Rückstellkraft ausschwenkbare Haltefeder (14), insbesondere Blattfeder vorgesehen ist und am distalen Ende des Führungsdrahtes (3) eine Querschnittsverminderung (15) angeordnet ist, in welche diese Feder (14) einrastet.

21. Vorrichtung nach Anspruch 20, dadurch gekennzeichnet, daß die als Trennhilfe im Inneren des Schlittens (4) angeordnete Blattfeder (14) an ihrem auslenkbaren Ende einen in Auslenkrichtung entgegen der Federkraft ragenden Vorsprung (16) hat, der bei ausgelenkter Feder in die Aufnahme-Ausnehmung (5) und dort in eine Ausnehmung des Elektrodenkopfes oder zwischen die Windungen einer Wendel (7) des Elektrodenkopfes paßt.

22. Vorrichtung nach einem der vorstehenden Ansprüche, dadurch gekennzeichnet, daß die Aufnahme-Ausnehmung (5) auf ihrer der Führungsöffnung (6) abgewandten Seite seitlich offen ist und die innere in Vorschubrichtung von dem Elektrodenkopf oder Katheterende beaufschlagte Wandung (17) dieser Aufnahme-Ausnehmung (5) gegen diese seitliche Öffnung (18) in Vorschubrichtung schräg verläuft und bei Druck auf die Elektrode oder den Katheter den Elektrodenkopf oder das Katheterende nach außen ablenkt.

23. Vorrichtung nach Anspruch 22, dadurch gekennzeichnet, daß nahe dem der stirnseitigen Schrägfläche (17) abgewandten Ende der Aufnahmeöffnung (5) wenigstens eine Klemme (19) oder Querschnittsverengung (20) zum Eingreifen in Ausnehmung oder Zwischenräume einer Wendel (7) des Elektrodenkopfes vorgesehen ist.

24. Vorrichtung nach Anspruch 22 oder 23, dadurch gekennzeichnet, daß die am Eintritt in die Aufnahme-Ausnehmung (5) vorgesehene Verengung (20) auch die seitliche Öffnung (18) der Aufnahme-Ausnehmung (5) teilweise übergreift und diese seitliche Öffnung (18) von dem hinteren Ende des Schlittens (4) her zunächst eine schmale, sich dann erweiternde Umrißform hat, wobei die Größe der Erweiterung wenigstens dem Umfang der Schraubwendel (7) oder des Elektrodenkopfes entspricht.

## Claims

1. A device for medical catheters (2) or electrodes (1) to enter a vein or artery by means of a guide wire (3), **characterized in that** the device has a separate slide (4) gliding on the guide wire (3) and that said slide (4) is provided with at least one female part (5) for receiving the front end of the catheter or of the electrode to be introduced.

2. A device as claimed in claim 1, characterized in that the slide (4) has at least one opening of passage or guide opening (6) for the guide wire (3), as well as a female part in the form of a recess (5) for surrounding the foremost end or front face of the catheter (2), of the electrode (1), of a mandrin or of a further guide wire.

3. A device as claimed in claim 1 or claim 2, characterized in that the receiving recess (5) is a blind hole in which a helical coil (7) of an electrode head fits.

4. A device as claimed in any one of claims 1 to 3, characterized in that the guide wire (3) has at the distal end thereof an enlargement (8), particularly a ball, having a cross section surpassing the cross section of the guide opening (6) which forms part of the slide (4) and engages over the guide wire (3).

5. A device as claimed in any one of claims 1 to 4, characterized in that at least one drawing element (8a) is applied to the slide (4), particularly to the rearward end thereof in the direction of entry.

6. A device as claimed in any one of claims 1 to 5, characterized in that the slide (4) tapers in the direction of entry, from the female part (5) for the electrode or catheter to be introduced towards the front mouth of the guide opening.

7. A device as claimed in any one of claims 1 to 6, characterized in that the rearward end of the slide (4) is rounded, bevelled or tapered in the area of the entrance to the guide opening (6) and preferably also to the female part (5).

8. A device as claimed in any one of claims 1 to 7, characterized in that the receiving recess (5) is arranged parallel to the guide opening (6) of the slide (4).

9. A device as claimed in any one of claims 1 to 8, characterized in that the entire slide (4) is traversed in the longitudinal direction or feed direction thereof by the receiving recess (5) which interiorly axially fixes clampingly or threadedly a helical coil (7) or the like during insertion, the helical coil (7) being adapted to be screwed into a wall of a body cavity or the like from the front end of said receiving recess (5).

10. A device as claimed in any one of the preceding claims, characterized in that the receiving recess in the interior of the slide (4) is stepped and a first zone (5a) has a larger cross section and a second zone (5b) forming part of said receiving recess (5) and opening out at the front end of the slide (4) in the direction of entry has a smaller cross section.

11. A device as claimed in any one of claims 1 to 10, characterized in that the guide opening (6) and the receiving recess (5) in each case have circular, closed cross sections.

12. A device as claimed in any one of the preceding claims, characterized in that the guide opening (6) and the receiving recess (5) have a corresponding cross section and serve alternately for guidance or for reception.

13. A device as claimed in any one of claims 1 to 12, characterized in that the slide (4) consists of slidable plastics and/or metal compatible to the body, particularly special steel.

14. A device as claimed in any one of claims 1 to 13, characterized in that the guide opening(s) (6) and/or receiving recess(es) is (are) lined with inert, particularly slidable plastics.

15. A device as claimed in any one of claims 1 to 14, characterized in that the slide (4) is adapted to be non-rotatably coupled to the guide thereof, in particular has two parallel guide openings (6) for simultaneously engaging two guide wires (3) and has the receiving recess (5) in the area outside said guide openings (6).

16. A device as claimed in any one of claims 1 to 15, characterized in that the slide (4) and/or the guide wire (3) has a device for aiding detachment which serves to fix the slide (4) at the distal end of the guide wire (3) and has a retention force opposing and exceeding the pulling force when the electrode head or the like is uncoupled.

17. A device as claimed in any one of claims 1 to 16, characterized in that the guide wire (3) and/or slide (4) is provided with a barb (10, 12) or coupling projection as a detachment aid, and that the barb (12) of the guide wire (3) has a stop (12a) for the slide (4) pushed over it and/or the barb (10) arranged on the slide itself has a hook projection (10a) locking into place in an annular groove (11) in the spherical enlargement (8) of the guide wire (3).

18. A device as claimed in any one of the preceding claims, characterized in that the barb-like detachment aid (10, 12) on the guide wire (3) and/or on the slide (4) consists of plastics.

19. A device as claimed in any one of claims 1 to 18, characterized in that in the form of a detachment or disengagement aid there is a tube (13) provided applied to the slide (4) at the end thereof to the rear in the direction of insertion, said tube fitting over the guide wire (3) and particularly being connected, e.g. adhesively secured, to the slide (4).

20. A device as claimed in any one of claims 1 to 19, characterized in that a retaining spring (14), particularly a leaf spring, is provided as a detachment or disengagement aid inside the slide (4), said spring being adapted to swing out through the guide wire (3) from the guide opening (6) into a recess situated within the slide against a restoring force and arranged at the distal end of the guide wire (3) is a reduction of cross-sectional area (15) which said spring (14) snaps into.

21. A device as claimed in claim 20, characterized in that the deflectable end of the leaf spring (14) arranged as a detachment aid inside the slide (4) has a projection (16) protruding in the direction of deflection counter to the spring tension, said projection fitting into the receiving recess (5) and there into a recess of the electrode head or between the turns of a coil (7) of the electrode head when the spring is deflected.

22. A device as claimed in any one of the preceding claims, characterized in that the receiving recess (5) is laterally open on the side thereof averted from the guide opening (6), and that inner wall (17) of said receiving recess (5) which is acted upon in the feed direction by the electrode head or catheter end shows an oblique development towards said lateral opening (18) in the feed direction, and outwardly deflects the electrode head or catheter end when pressure is exerted on the electrode or catheter.

23. A device as claimed in claim 22, characterized in that provision is made for at least one clip (19) or reduction of cross section (20) which serves to engage in recesses or interspaces of a coil (7) of the electrode head and is near that end of the receiving opening (5) which is averted from the front oblique face (17).

24. A device as claimed in claim 22 or claim 23, characterized in that the constriction (20) provided at the entrance to the receiving recess (5) also partly covers the lateral opening (18) of the receiving recess (5), and from the rear end of the slide (4) said lateral opening (18) first has a narrow, then widening outer contour, the size of the widening corresponding at least to the periphery of the helical coil (7) or the periphery of the electrode head.

## Revendications

1. Dispositif pour l'introduction transveineuse ou artérielle de cathéters (2) ou d'électrodes (1) médicaux au moyen d'un fil guide (3), caractérisé en ce qu'il comporte un coulisseau séparé (4) glissant sur le fil guide (3) et que ce coulisseau (4) est pourvu d'au moins une réception (5) pour l'extrémité antérieure du cathéter ou de l'électrode à introduire.

2. Dispositif selon la revendication 1, caractérisé en ce que le coulisseau (4) possède au moins une ouverture de passage ou de guidage (6) ainsi que, en tant que réception (5), un évidement récepteur conformé pour entourer l'extrémité antérieure ou le côté frontal du cathéter (2), de l'électrode (1), d'un mandrin ou d'un fil guide supplémentaire.

3. Dispositif selon la revendication 1 ou 2, caractérisé en ce que l'évidement récepteur (5) est un trou borgne dans lequel s'ajuste une hélice à visser (7) d'une tête d'électrode.

4. Dispositif selon une des revendications 1 à 3, caractérisé en ce que le fil guide (3) possède un épaississement (8) à son extrémité distale, en particulier une boule, dont la section est supérieure à la section de l'ouverture de guidage (6) par laquelle le coulisseau (4) est enfilé sur le fil guide (3).

5. Dispositif selon une des revendications 1 à 4, caractérisé en ce qu'au moins un élément de tirage (8a) attaque le coulisseau (4), en particulier à son extrémité postérieure dans la direction d'introduction.

6. Dispositif selon une des revendications 1 à 5, caractérisé en ce que le coulisseau (4) se rétrécit dans la direction d'introduction, depuis la réception (5) pour l'électrode ou le cathéter à introduire vers l'embouchure antérieure de l'ouverture de guidage.

7. Dispositif selon une des revendications 1 à 6, caractérisé en ce que l'extrémité postérieure du coulisseau (4) est arrondie, biseautée ou amincie dans la région de l'entrée de l'ouverture de guidage (6) et de préférence aussi de la réception (5).

8. Dispositif selon une des revendications 1 à 7, caractérisé en ce que l'évidement récepteur (5) est parallèle à l'ouverture de guidage (6) du coulisseau (4).

9. Dispositif selon une des revendications 1 à 8, caractérisé en ce que l'évidement récepteur (5) traverse tout le coulisseau (4) dans le sens de sa longueur, correspondant à la direction d'avancement, et fixe axialement, à l'intérieur de lui, par serrage ou au moyen d'un filetage, une hélice à visser (7) ou un élément semblable pendant l'introduction, l'hélice (7) pouvant être vissée hors de l'extrémité antérieure de l'évidement récepteur (5) et dans une paroi d'une cavité corporelle ou analogue.

10. Dispositif selon une des revendications précédentes, caractérisé en ce que l'évidement récepteur est étagé à l'intérieur du coulisseau (4) et possède une première zone (5a) de plus grande section droite et une seconde zone (5b) de cet évidement récepteur (5), qui débouche à l'extrémité antérieure du coulisseau (4) dans la direction d'introduction, et présente une plus petite section droite.

11. Dispositif selon une des revendications 1 à 10, caractérisé en ce que l'ouverture de guidage et l'évidement récepteur possèdent chacun une section droite circulaire fermée.

12. Dispositif selon une des revendications précédentes, caractérisé en ce que l'ouverture de guidage (6) et l'évidement récepteur (5) ont des sections droites concordantes et servent alternativement au guidage et à la réception.

13. Dispositif selon une des revendications 1 à 12, caractérisé en ce que le coulisseau (4) est réalisé d'une matière plastique douée de caractéristiques de glissement et/ou d'un métal, en particulier d'acier fin, toléré par l'organisme.

14. Dispositif selon une des revendications 1 à 13, caractérisé en ce que l'ouverture de guidage ou les ouvertures de guidage (6) et/ou l'évidement récepteur ou les évidements récepteurs est ou sont garni(s) d'une matière plastique inerte, douée en particulier de caractéristiques de glissement.

15. Dispositif selon une des revendications 1 à 14, caractérisé en ce que le coulisseau (4) peut être accouplé sans rotation possible à son guide et possède en particulier deux ouvertures de guidage (6) parallèles pour le maintien simultané sur deux fils guides (3), l'évidement récepteur (5) étant prévu dans la zone située à l'extérieur de ces ouvertures de guidage (6).

16. Dispositif selon une des revendications 1 à 15, caractérisé en ce que le coulisseau (4) et/ou le fil guide comporte un dispositif auxiliaire de séparation pour immobiliser le coulisseau (4) à l'extrémité distale du fil guide (3), dispositif qui possède une force de maintien opposée et supérieure à la force de traction appliquée pour désaccoupler la tête d'électrode ou analogue.

17. Dispositif selon une des revendications 1 à 16, caractérisé en ce qu'un crochet (10, 12) ou une saillie de couplage est prévu en tant que dispositif auxiliaire de séparation sur le fil guide (3) et/ou sur le coulisseau (4), et que, s'il est prévu sur le fil guide (3), le crochet (12) présente une butée (12a) pour le coulisseau (4) lorsque celui-ci a été poussé pardessus et au-delà du crochet et/ou que le crochet (10) prévu sur le coulisseau lui-même, présente une saillie crochue (10a) destinée à 's'encliqueter dans une gorge annulaire (11) de l'épaississement (8) en forme de boule du fil guide (3).

18. Dispositif selon une des revendications précédentes, caractérisé en ce que le dispositif auxiliaire de séparation (10, 12) en forme de crochet sur le fil guide (3) et/ou sur le coulisseau (4) est en matière plastique.

19. Dispositif selon une des revendications 1 à 18, caractérisé en ce qu'un tuyau souple (13), pouvant être enfilé par-dessus le fil guide (3) et attaquant l'extrémité postérieure du coulisseau (4) dans la direction d'introduction, est prévu en tant que dispositif auxiliaire de séparation ou de détachement, tuyau qui est en particulier relié au coulisseau (4), par collage par exemple.

20. Dispositif selon une des revendications 1 à 19, caractérisé en ce qu'un ressort d'arrêt (14), en particulier un ressort plat, est prévu en tant que dispositif auxiliaire de séparation ou de détachement à l'intérieur du coulisseau (4), ressort que le fil guide (3) est capable de faire fléchir à l'encontre d'une force de rappel hors de l'ouverture de guidage (6) et dans une découpe prévue à l'intérieur du coulisseau, et l'extrémité distale du fil guide (3) présente une réduction de section (15) dans laquelle vient s'encliqueter ce ressort (14).

21. Dispositif selon la revendication 20, caractérisé en ce que le ressort plat (14), logé en tant que dispositif auxiliaire de séparation à l'intérieur du coulisseau (4), possède, à son extrémité pouvant être déviée, une saillie (16) orientée dans la direction de déviation et dirigée en sens contraire à la force du ressort, saillie qui pénètre dans l'évidement récepteur (5) lorsque le ressort est dévié et s'ajuste à l'intérieur de cet évidement dans un ajour de la tête d'électrode ou entre les spires d'une hélice (7) de la tête d'électrode.

22. Dispositif selon une des revendications précédentes, caractérisé en ce que l'évidement récepteur (5) est latéralement ouvert sur son côté éloigné de l'ouverture de guidage (6) et la paroi intérieure (17) de cet évidement récepteur (5), sur laquelle la tête d'électrode ou l'extrémité du cathéter agit dans la direction d'avancement, s'étend obliquement vers cette ouverture latérale (18), dans la direction d'avancement, et provoque la déviation vers l'extérieur de la tête d'électrode ou de l'extrémité du cathéter lorsqu'une pression est exercée sur l'électrode ou le cathéter.

23. Dispositif selon la revendication 22, caractérisé en ce qu'au moins une pince (19) ou un rétrécissement de section (20) est prévu près de l'extrémité de l'évidement récepteur (5) éloignée de la face oblique (17) située du côté frontal, pince ou rétrécissement qui est destiné à pénétrer dans un ajour ou des intervalles d'une hélice (7) de la tête d'électrode.

24. Dispositif selon la revendication 22 ou 23, caractérisé en ce que le rétrécissement (20), prévu à l'entrée de l'évidement récepteur (5), ferme également en partie l'ouverture latérale (18) de cet évidement (5), et l'ouverture latérale (18) possède un contour ayant d'abord une forme étroite, à partir de l'extrémité postérieure du coulisseau (4), et s'élargissant ensuite à une dimension correspondant au moins à la largeur de l'hélice à visser (7) ou de la tête d'électrode.

Fig.1

EP 0 348 692 B1

Fig.2

EP 0 348 692 B1

Fig.3

8a  1(3)  4  7  5  7  8

3

6

Pf 1

EP 0 348 692 B1

Fig.4

EP 0 348 692 B1

Fig.5

Fig.6

Fig.7

Pf1

EP 0 348 692 B1

Fig. 8

Fig. 9

Fig. 10

*Fig. 11*

EP 0 348 692 B1

Fig. 12

Fig. 13

1
5
7
4
14
16
3
Pf5
6
15

1
Pf4
5
16
4
7
Pf3
8
3
14
6
15

EP 0 348 692 B1

Fig. 14

Fig. 15

Fig. 16

Fig. 17

EP 0 348 692 B1